# EUROPÄISCHE PATENTSCHRIFT

(11) **EP 2 194 178 B1**
(45) Veröffentlichungstag und Bekanntmachung des Hinweises auf die Patenterteilung: **26.03.2014**
(21) Anmeldenummer: 09014534.3
(22) Anmeldetag: 21.11.2009
(51) Int. Cl.: D04H 13/00, A61F 13/56

(54) **Verbundmaterial**
Compound material
Matériau composite

(30) Priorität: 06.12.2008 DE 202008016226 U
(43) Veröffentlichungstag der Anmeldung: 09.06.2010
(73) Patentinhaber: Mondi Consumer Packaging Technologies GmbH, 48599 Gronau (DE)
(72) Erfinder: Baldauf, Georg, Dipl.-Ing., 48366 Laer (DE); Homölle, Dieter, Dipl.-Ing. Chem., 48607 Ochtrup (DE); Schönbeck, Marcus, Dipl.-Ing., 33775 Versmold (DE)
(74) Vertreter: Albrecht, Rainer Harald

(56) Entgegenhaltungen:
- EP-A1- 1 295 711
- EP-A1- 1 686 209
- EP-A1- 1 736 306
- EP-A1- 1 918 092
- EP-A1- 1 997 942
- EP-A1- 2 110 241
- US-A1- 2006 068 168

## Beschreibung

Die Erfindung betrifft ein Verbundmaterial insbesondere zur Fertigung von flexiblen Windelverschlusselementen. Das Verbundmaterial weist Außenschichten aus Nonwoven und zwischen den Außenschichten einkaschierten parallelen Streifen aus einer elastischen Folie auf. Dabei sind die Außenschichten in den Abschnitten zwischen den elastischen Folienstreifen unmittelbar miteinander verbunden. Diese Abschnitte bilden nicht elastische Bereiche des Verbundmaterials. Außerdem bilden die jeweils einen Folienstreifen enthaltenden Abschnitte des Verbundmaterials elastische Bereiche.

Ein gattungsgemäßes Verbundmaterial ist aus der EP 1 686 209 A1 bekannt. Das in dem dort beschriebenen Verfahren gebildete Verbundmaterial weist elastische und nicht elastische Bereiche auf, wobei parallele Streifen aus elastischem Laminat im Abstand zueinander zwischen zwei Bahnen aus Nonwoven einkaschiert werden. Nachteilig bei dem dort beschriebenen Verbundmaterial ist, dass die elastischen Bereiche eine andere Lichtdurchlässigkeit aufweisen als die nicht elastischen Bereiche. Somit erscheinen bei der Verwendung des Verbundmaterials, beispielsweise bei einer Babywindel, die elastischen Bereiche heller als die nicht elastischen Bereiche. Auf diese Weise entsteht der Eindruck, das Verbundmaterial, welches sichtbarer Bestandteil eines Windelverschlusses ist, sei aus uneinheitlichen Materialien hergestellt worden. Der Benutzer des Windelverschlusses zieht daraus womöglich die Schlussfolgerung, das Verschlusselement sei instabil, wodurch der Windelverschluss insgesamt vom Benutzer minderwertig eingeschätzt wird.

Vor diesem Hintergrund liegt der Erfindung die Aufgabe zugrunde, ein Verbundmaterial anzugeben, welches bei dem Benutzer den Eindruck hinterlässt, dass das Verbundmaterial einheitlich gefertigt wurde.

Die Aufgabe wird erfindungsgemäß, dadurch gelöst, dass zumindest eine der aus einem durchscheinenden Nonwoven bestehenden Außenschichten des Verbundmaterials mit einem Muster aus Wellenlinien, die sich quer zur Dehnungsrichtung des Verbundmaterials erstrecken, bedruckt ist, wobei der Aufdruck sich über die elastischen und nicht elastischen Bereiche erstreckt. Durch diesen Aufdruck wird der Unterschied der Lichtdurchlässigkeit zwischen elastischem und nicht elastischem Bereich des Verbundmaterials reduziert. Beide Bereiche sind weniger durchscheinend und der Benutzer des Verbundmaterials erhält den Eindruck, es sei nicht durch Aneinanderfügen unterschiedlich beschaffener Materialkomponenten hergestellt. Insbesondere dann, wenn das Verbundmaterial mit einem Muster bedruckt ist, wird das einheitliche Erscheinungsbild verstärkt. Durch die optische Angleichung von elastischen und unelastischen Bereichen des Verbundmaterials wird bei dem Benutzer ein größeres Vertrauen in die Haltbarkeit und in die qualitative Beschaffenheit des Verbundmaterials erzeugt. Bei den verwendeten Materialien kann es sich beispielsweise um ein gardiertes Nonwoven mit einem Flächengewicht von 20 bis 35 g/m² (Gramm pro Quadratmeter) oder um ein mehrlagiges Nonwoven handeln. Geeignet ist insbesondere ein mehrlagiges Nonwoven mit einer Lage aus schmelzgeblasenen (melt blown) Nonwoven zwischen zwei Lagen aus spinngebundenen (spunbond) Nonwoven. Dieses als SMS-Nonwoven bezeichnete Material weist vorzugsweise ein Flächengewicht zwischen 10 und 30 g/m² auf. Die in den elastischen Bereichen einkaschierte Folie kann eine Stärke von 20 bis 120 µm aufweisen. Die Kaschierung des Nonwovens mit der elastischen Folie kann vorzugsweise durch ein Holtmelt-Kaschierverfahren erfolgen.

Vorteilhaft bei der erfindungsgemäßen Ausgestaltung des Musters ist, dass dieses auch bei Dehnung des Verbundmaterials als erkennbares Muster erhalten bleibt. Darüber hinaus bietet beispielsweise eine wellenförmige Gestaltung des Musters dem Benutzer die Möglichkeit, beispielsweise einen Windelverschluss immer um das gleiche Maß zu dehnen, in dem sich bei dem Benutzer die Gestalt des durch die Dehnung veränderten Musters einprägt. Indirekt bildet ein solches Muster somit eine Skala für die Dehnung.

Entsprechend kann in einer bevorzugten Ausgestaltung auch vorgesehen sein, dass Wellen vorgesehen sind, die in ungedehntem Zustand in den elastischen Bereichen einerseits und den unelastischen Bereichen andererseits einen unterschiedlichen Abstand und/oder unterschiedliche Ausdehnungen die Dehnungsrichtung aufweisen, wobei erst bei Erreichen einer für die Benutzung bevorzugten Dehnung ein gleichmäßiges Muster gebildet wird.

Bei einer weiteren Ausgestaltung des Verbundmaterials kann zudem vorgesehen sein, dass der Aufdruck an der Innenfläche der Nonwovenschicht angeordnet ist. Durch diese Anordnung des Aufdrucks wird vermieden, dass Bestandteile des Druckmaterials, etwa Farbe, beispielsweise bei Verwendung des Verbundmaterials bei einem Windelverschluss mit der Haut des Windelträgers in Berührung kommt. Dies kann insbesondere bei der Vorbeugung zur Vermeidung von ungewünschten Hautirritationen zweckmäßig sein.

Eine weitere Ausgestaltung des Verbundmaterials sieht vor, dass der Aufdruck auf einer vorbehandelten Fläche der Nonwovenschicht angeordnet ist. Die Vorbehandlung der Fläche besteht dabei aus einem Auftrag eines Vliesprimers, der einen Farbdurchschlag durch das Nonwoven weitgehend verhindert. Dies bietet insbesondere bei der Herstellung des erfindungsgemäßen Verbundmaterials insofern einen Vorteil als die Maschine, welche das Verbundmaterial herstellt, bei einer Verhinderung eines Farbdurchschlags weniger oder gar nicht verschmutzt wird. Der Herstellungsprozess kann dadurch auch beschleunigt ab-laufen. Es kann außerdem vorgesehen sein, dass der Aufdruck aus einem im Tiefdruckverfahren auf die Nonwovenschicht aufgebrachten Druckbild besteht.

Zur Erzeugung des Druckbildes können lösemittelbasierte, wasserbasierte oder auch UV-härtende Druckfarben eingesetzt werden. Insbesondere kann auch vorgesehen sein, dass die Druckfarbe für den Aufdruck auf einem Polyvinylbutyral (PVB), einem Zellulosenitrat (NC) oder auf einer Mischung aus Zellulosenitrat und Polyamid (NC/PA) basiert und vorzugsweise mit einem Haftharz versetzt ist. Die Bedruckung kann dabei inline durchgeführt werden, das heißt, dass das Verbundmaterial zunächst bedruckt wird, dann die Folienstreifen geschnitten werden und anschließend eine dreilagige Streifen-Holtmeltkaschierung durchgeführt wird. Das Bedrucken stellt demnach bei der Herstellung des erfindungsgemäßen Verbundmaterials einen in dem gesamten Herstellungsverfahrensablauf leicht zu integrierenden zusätzlichen Verfahrensschritt dar.

Es kann darüber hinaus bei dem erfindungsgemäßen Verbundmaterial vorgesehen sein, dass die elastischen Bereiche des Verbundmaterials durch eine Überdehnung mechanisch aktiviert sind, wobei die Außenschicht vorzugsweise vor der mechanischen Aktivierung bedruckt worden ist. Diese mechanische Aktivierung bemisst eine teilweise Zerfaserung der nicht elastischen Außenschichten, wodurch eine leichte Dehnbarkeit des Verbundmaterials in den elastischen Bereichen erreicht wird.

Die Erfindung wird im Folgenden anhand einer lediglich ein Ausführungsbeispiel darstellenden Zeichnung erläutert. Die Figuren zeigen:
- **Fig. 1**: eine Aufsicht auf einen Abschnitt des Verbundmaterials und
- Fig. 2: einen vertikalen Schnitt durch das Verbundmaterial.

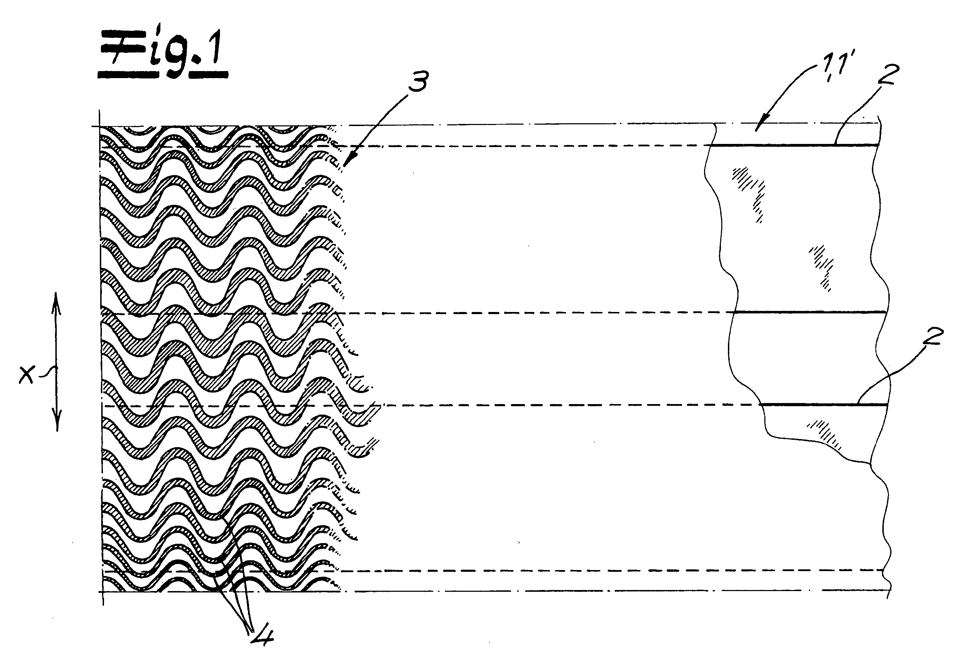

Fig. 1 zeigt eine Aufsicht auf ein Verbundmaterial, insbesondere zur Fertigung von flexiblen Windelverschlusselementen, mit Außenschichten 1, 1' aus Non-woven und zwischen den Außenschichten 1, 1' einkaschierten parallelen Streifen 2 aus einer elastischen Folie. Die Außenschichten 1, 1' sind dabei in den Abschnitten zwischen den elastischen Folienstreifen 2 unmittelbar miteinander verbunden. Sie bilden nicht elastische Bereiche des Verbundmaterials. Die jeweils einen Folienstreifen 2 enthaltenden Abschnitte des Verbundmaterials bilden dabei die elastischen Bereiche. In der Fig. 1 ist zu erkennen, dass zumindest eine der aus einem durchscheinenden Nonwoven bestehenden Außenschichten 1, 1' farbig bedruckt ist, wobei der Aufdruck 3 sich über die elastischen und nicht elastischen Bereiche erstreckt. Gemäß dem dargestellten Ausführungsbeispiel weist eine der Außenschichten 1, 1' einen Aufdruck 3 in Form eines Muster auf. Durch dieses Muster erscheinen die elastischen und unelastischen Bereiche einander ähnlicher. Ein Unterschied hinsichtlich der Lichtdurchlässigkeit oder des optischen Erscheinungsbildes wird verringert. Wie der Fig. 1 zu entnehmen ist, besteht das Muster erfindungsgemäß aus Wellenlinien 4, die sich quer zur Dehnungsrichtung x des Verbundmaterials erstrecken. Indem sich das Muster quer zur Dehnungsrichtung x des Materials erstreckt, bleibt die Gestalt bzw. die Form des Musters auch nach einer Dehnung des Verbundmaterials im Wesentlichen erhalten. Ein durch Dehnung verzerrtes Wellenmuster in einem der elastischen Bereiche unterscheidet sich nur unwesentlich von dem Wellenmuster des unelastischen, nicht dehnbaren Bereichs des Verbundmaterials. Bei dem in Fig. 1 dargestellten Ausführungsbeispiel des erfindungsgemäßen Verbundmaterials kann es zudem zweckmäßig sein, dass der Aufdruck 3 an der Innenfläche der Außenschicht 1 angeordnet ist, die bei einer Verwendung des Verbundmaterials als Teil eines Windelverschlusselementes über den Folienstreifen 2 angeordnet ist.

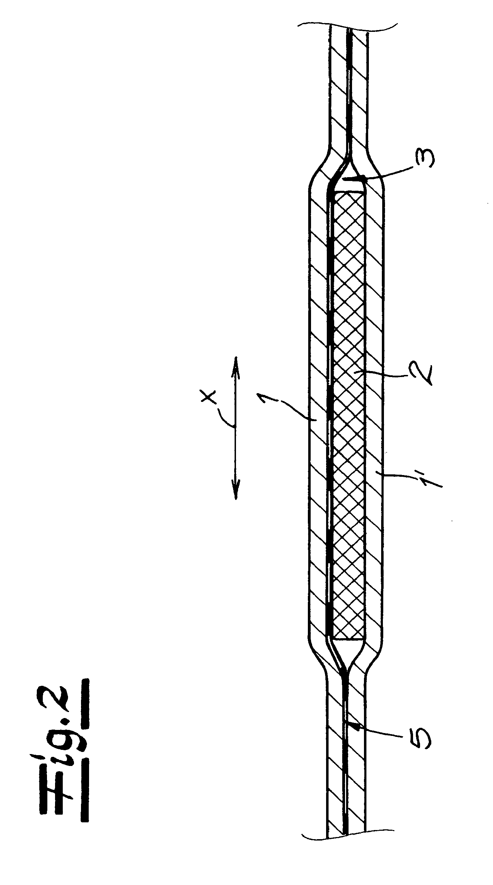

Fig. 2 zeigt einen vertikalen Schnitt durch das erfindungsgemäße Verbundmaterial entlang der Dehnungsrichtung x, wobei der Aufdruck 3 auf einer vorbehandelten Fläche 5 der in der Fig. 2 dargestellten oberen Außenschicht 1 an der Innenseite angeordnet ist und wobei die Vorbehandlung aus dem Auftrag eines Vliesprimers besteht, der einen Farbdurchschlag durch das Nonwoven weitgehend verhindert. Es kann zudem vorgesehen sein, dass der Aufdruck 3 aus einem im Tiefdruckverfahren auf die Nonwovenschicht aufgebrachten Druckbild besteht. Die Druckfarbe für den Aufdruck 3 kann dabei auf einem Polyvinylbutyral (PVB), Zellulosenitrat (NC) oder einer Mischung aus Zellulosenitrat und Polyamid (NC/PA) basieren. Vorzugsweise kann die Druckfarbe mit einem Haftharz versetzt sein. Es kann zudem auch vorgesehen sein, dass die elastischen Bereiche des Verbundmaterials durch eine Überdehnung mechanisch aktiviert sind, wobei die Außenschicht 1 vor der mechanischen Aktivierung bedruckt worden ist.

## Patentansprüche

1. Verbundmaterial, insbesondere zur Fertigung von flexiblen Windelverschlusselementen, mit Außenschichten (1, 1 ') aus Nonwoven und zwischen den Außenschichten (1, 1') einkaschierten parallelen Streifen (2) aus einer elastischen Folie,
wobei die Außenschichten (1, 1') in den Abschnitten zwischen den elastischen Folienstreifen (2) unmittelbar miteinander verbunden sind und nicht elastische Bereiche des Verbundmaterials bilden und
wobei die jeweils einen Folienstreifen (2) enthaltenden Abschnitte des Verbundmaterials elastische Bereiche bilden,
**dadurch gekennzeichnet, dass** zumindest eine der aus einem durchscheinenden Nonwoven bestehenden Außenschichten (1, 1') mit einem Muster aus Wellenlinien (4), die sich quer zur Dehnungsrichtung (x) des Verbundmaterials erstrecken, bedruckt ist, wobei der Aufdruck (3) sich über die elastischen und nicht elastischen Bereiche erstreckt.

2. Verbundmaterial nach Anspruch 1, **dadurch gekennzeichnet, dass** der Aufdruck (3) im gedehnten Zustand ein gleichmäßiges Muster bildet.

3. Verbundmaterial nach Anspruch 1 oder 2, **dadurch gekennzeichnet, dass** der Aufdruck (3) an der Innenfläche einer der Außenschichten (1) angeordnet ist.

4. Verbundmaterial nach einem der Ansprüche 1 bis 3, **dadurch gekennzeichnet, dass** der Aufdruck (3) auf einer vorbehandelten Fläche (5) einer der Außenschichten (1) angeordnet ist und dass die Vorbehandlung aus dem Auftrag eines Vliesprimers besteht, der einen Farbdurchschlag durch das Non-woven weitgehend verhindert.

5. Verbundmaterial nach einem der Ansprüche 1 bis 4, **dadurch gekennzeichnet, dass** der Aufdruck (3) aus einem im Tiefdruckverfahren aufgebrachten Druckbild besteht.

6. Verbundmaterial nach einem der Ansprüche 1 bis 5, **dadurch gekennzeichnet, dass** die Druckfarbe für den Aufdruck (3) auf einem Polyvinylbutyral (PVB), Zellulosenitrat (NC) oder eine Mischung aus Zellulosenitrat und Polyamid (NC/PA) basiert und vorzugsweise mit einem Haftharz versetzt ist.

7. Verbundmaterial nach einem der Ansprüche 1 bis 6, **dadurch gekennzeichnet, dass** die elastischen Bereiche des Verbundmaterials durch eine Überdehnung mechanisch aktiviert sind, wobei zumindest eine der Außenschichten (1) vor der mechanischen Aktivierung bedruckt worden ist.

## Claims

1. A composite material, in particular for manufacturing flexible nappy closure elements, with outer layers (1, 1') made of non-woven and parallel strips (2) consisting of an elastic film laminated between the outer layers (1, 1'),
wherein the outer layers (1, 1') are directly connected with each other in the sections between the elastic film strips (2) and form non-elastic areas of the composite material, and
wherein the sections of the composite material respectively containing a film strip (2) form elastic areas,
**characterised in that** at least one of the outer layers (1, 1') consisting of a translucent non-woven is imprinted with a pattern of wavy lines (4) extending transversely to the stretching direction (x) of the composite material, wherein the imprint (3) extends across the elastic and the non-elastic areas.

2. The composite material according to claim 1, **characterised in that** the imprint (3) forms a regular pattern when in a stretched state.

3. The composite material according to claim 1 or 2, **characterised in that** the imprint (3) is arranged on the inner surface of one of the outer layers (1).

4. The composite material according to one of claims 1 to 3, **characterised in that** the imprint (3) is arranged on a pre-treated surface (5) of one of the outer layers (1) and that pre-treatment consists of applying a fleece primer which substantially prevents seepage through the non-woven.

5. The composite material according to one of claims 1 to 4, **characterised in that** the imprint (3) consists of a printed image applied by way of intaglio printing.

6. The composite material according to one of claims 1 to 5, **characterised in that** the printing ink for the imprint (3) is based on a polyvinyl butyral (PVB), on cellulose nitrate (NC) or a mixture of cellulose nitrate and polyamide (NC/PA) and preferably has an adhesion resin added to it.

7. The composite material according to one of claims 1 to 6, **characterised in that** the elastic areas of the composite material are mechanically activated by overstretching, wherein at least one of the outer layers (1) has been imprinted prior to mechanical activation.

## Revendications

1. Matière composite, notamment pour la fabrication d'éléments souples pour la fermeture de couches-culottes avec des couches extérieures (1, 1') en non-tissé et avec des rubans (2) parallèles en un film élastique, contrecollés entre les couches extérieures (1, 1'),
dans les parties entre les rubans (2) élastiques en film, les couches extérieures (1, 1') étant directement reliées les unes aux autres et formant des zones non élastiques de la matière composite et
les parties de la matière composite contenant chacune un ruban (2) de film formant des zones élastiques,
**caractérisée en ce qu'**au moins l'une des couches extérieures (1, 1') consistant dans un non-tissé transparent est imprimée d'un motif en lignes ondulées (4), qui s'étendent à la transversale du sens d'extension (x) de la matière composite, l'impression (3) s'étendant sur les zones élastiques et non élastiques.

2. Matière composite selon la revendication 1, **caractérisée en ce qu'**à l'état étiré, l'impression (3) forme un motif régulier.

3. Matière composite selon la revendication 1 ou 2, **caractérisée en ce que** l'impression (3) est placée sur la surface interne de l'une des couches extérieures (1).

4. Matière composite selon l'une quelconque des revendications 1 à 3, **caractérisée en ce que** l'impression (3) est placée sur une surface (5) prétraitée de l'une des couches extérieures (1) et **en ce que** le prétraitement consiste dans l'application d'un apprêt primaire de non-tissé qui empêche largement une remontée d'encre à travers le non-tissé.

5. Matière composite selon l'une quelconque des revendications 1 à 4, **caractérisée en ce que** l'impression (3) consiste dans image imprimée appliquée par procédé d'héliogravure.

6. Matière composite selon l'une quelconque des revendications 1 à 5, **caractérisée en ce que** l'encre d'impression pour l'impression (3) est basée sur un butyral de polyvinyle (PVB), sur une nitrocellulose (NC) ou sur un mélange de nitrocellulose et de polyamide (NC/PA) et est mélangée de préférence à une résine adhésive.

7. Matière composite selon l'une quelconque des revendications 1 à 6, **caractérisée en ce que** les zones élastiques de la matière composite sont mécaniquement activées par un allongement excessif, au moins l'une des couches extérieures (1) ayant été imprimée avant l'activation mécanique.
